# EUROPEAN PATENT SPECIFICATION

(11) **EP 0 818 956 B1**
(45) Date of publication and mention of the grant of the patent: **08.10.2003**
(21) Application number: 95915851.0
(22) Date of filing: 04.04.1995
(51) Int. Cl.: A23L 1/10, A23L 1/164, A23L 1/308, A23L 1/18

(54) **CEREAL FOOD PRODUCT HIGH IN SOLUBLE FIBER**
CEREALIEN ENTHALTENDES LEBENSMITTEL MIT EINEM HOHEN GEHALT AN LÖSLICHEN BALLASTSTOFFEN
PRODUIT CEREALIER RICHE EN FIBRES SOLUBLES

(43) Date of publication of application: 21.01.1998
(73) Proprietor: SOCIETE DES PRODUITS NESTLE S.A., 1800 Vevey (CH)
(72) Inventor: WURSCH, Pierre, CH-1814 La Tour-de-Peilz (CH); FAYARD, Gilles, CH-1052 Le Mont-sur-Lausanne (CH); BURRI, Josef, CH-1066 Epalinges (CH)
(86) International application number: EP9501256
(87) International publication number: WO96031128

(56) References cited:
- WO-A-94/28743
- US-A- 4 497 840
- US-A- 5 169 662
- FOOD PROCESSING, USA, 1992, 53_(7) 84, 86, LABELL, F. 'Oat fiber has high beta-glucans level.'
- TRENDS IN FOOD SCIENCE & TECHNOLOGY, 1991, 2_(12) 311-314, WOOD, P. 'Oat BETA-glucan - physicochemical properties and physiological effects.'
- CARBOHYDR. POLYM., 1994, 331-6, WOOD, PETER J. 'Evaluation of oat bran as a soluble fiber source. Characterization of oat.beta.-glucan and its effects on glycemic response'
- CEREAL CHEMISTRY, vol. 70, no. 6, 1993 pages 712-715, W.-M. WANG AND C.F.KLOPFENSTEIN 'Effect of Twin-Screw Extrusion on the Nutritional Quality of Wheat, Barley, and Oats'

## Description

### Field of the Invention

This invention relates to a cereal-based, food product which is high in dietary fiber; particularly soluble fiber, and which is in ready to eat form. The food product is particularly useful for lowering blood lipid, cholesterol, and glucose levels, and as a food for diabetics. The invention also relates to methods of reducing glycemic levels in plasma.

### Background to the invention

A diet which is high in dietary fiber has many commonly accepted advantages or benefits. These advantages include normalization of bowel function, reduction of occurrence of certain colonic diseases, lowering of blood glucose concentrations, lowering of postprandial insulin levels, lowering of plasma cholesterol levels, decreasing blood lipid levels, and the like. For these reasons, diets high in soluble fibers are generally recommended by health authorities.

Diets high in dietary fibers are also strongly recommended for diabetics; for example Anderson, J.W. and Akanji, A.O.; 1993; "Treatment of Diabetes with High Fiber Diets", CRC Handbook of Dietary Fiber in Human Nutrition, CRC Press Inc, 2nd Edition, pages 443 to 470 indicate that these diets can reduce insulin requirements by up to 50% and improve glycemic control in type I diabetes. No significant long-term nutritional risks or side effects have been reported for diets high in dietary fibers.

Despite all the advantages and benefits of diets high in dietary fiber, in many industrialized societies, the average daily intake of dietary fiber is unfortunately much less than the recommended amount; often as little as half of the recommended amount. One reason for this is that convenient food products which are high in dietary fiber usually do not have an acceptable texture and mouthfeel. In particular such food products are usually very dry in the mouth. Alternatively the products are not convenience foods and require long preparation time; for example porridges.

Dietary fibers are usually characterized as water soluble or water insoluble. Anderson and Akanji (1993), among others, report that the water soluble fibers (for example guar, pectin, and psyllium) reduce, to an extent greater than insoluble fibers, the glycemic response to food. This makes food products which contain soluble fibers particularly suitable for diabetics. Further many of the advantages discussed above of a diet high in dietary fiber can be primarily attributed to the soluble fiber component. This has been confirmed in Braaten *et al*; 1994; Diabetic Medicine, **11,** 312 to 318 which describes a test in which foods rich in soluble fibre derived from oats significantly reduced glucose and insulin responses in plasma. However, the foods were administered in the form of a porridge. A porridge may be acceptable for clinical trials but is not a convenience food and hence not a good medium in which to present the soluble fibre.

Unfortunately, and as discussed in US patent 5,024,996, convenience food products which contain high levels of soluble fiber are very difficult to manufacture. One reason set forth in US patent 5,024,996 is due to the soluble fiber absorbing undesirably high amounts of moisture during processing. Also as discussed in US patent 5,024,996, products containing high levels of soluble fiber usually have poor texture and mouthfeel. In particular, the products have a gummy, slimy texture; especially when combined with milk which is often the case for cereals. The problems are reported to be especially heightened if the soluble fiber is derived from oat bran. The attempt to deal with the problem described in US patent 5,024,996 comprises using barley bran as a primary source of soluble fiber; there being at least as much soluble fiber as insoluble fiber but a minimum of 10% soluble fiber. If oat flour or bran is included, it is included primarily as a starchy component although it is acknowledged that oat bran may contain up to 10% soluble fiber and may contribute to the soluble fiber provided by the barley bran.

Another attempt to provide an acceptable convenience food product containing purportedly high levels of soluble fiber is described in US patent 5,169,662. Here conventional oat bran is combined with corn bran and corn meal and then extrusion-cooked to provide an expanded food product. The patent mentions that "it has been determined that an expanded oat bran product cannot be produced by using oat bran alone" and for this reason corn bran and the corn meal is added. However the corn bran and corn meal has the effect of substantially reducing the soluble fiber content of the food product; contrary to what is stated in the patent. Although US patent 5,169,662 describes the corn bran as having about 56% soluble fiber, this is at least 10 times higher than the level usually attributed to corn bran. In particular, the very report (Prosky et al; 1988; J. Assoc. Off. Anal. Chem, **70**, 5, 1017) which is referred to in the patent as describing the test method for determining soluble fiber, describes corn bran to have less than 1% soluble fiber. Therefore it appears that US patent 5,169,662 is in error concerning the soluble fiber content of the product. Consequently it is not surprising that a texturally acceptable product may have been obtained since such low levels of soluble fiber were present.

Further, at least part of the effectiveness of the soluble fiber resides in the ability of the soluble fiber to swell and increase the viscosity of the contents of the stomach and the small intestine (Edwards et al; 1987; Am J. Clin. Nutrition., **46**, 72 to 77). Unfortunately cooking during processing of products which contain soluble fibers may lead to some degrading of the soluble fibers such that their ability to increase viscosity is diminished.

Therefore there is still a need for an organoleptically acceptable, convenience food product which contains high levels of soluble fiber and which is able to increase the viscosity of the contents of the stomach and small intestine.

### Summary of the Invention

Accordingly this invention provides an extrusion cooked cereal product in ready-to-eat form, the cereal product containing a defatted oat bran concentrate which has a β-glucan content of at least 10% by weight; the cereal product having a β-glucan content of at least 5% by weight and in solution providing a viscosity at least as great as the viscosity provided by the components of the cereal product prior to being extrusion cooked.

Surprisingly, it has been found that a ready-to-eat product which has excellent texture and organoleptic properties, in particular mouthfeel, may be produced, despite the product containing high levels of soluble fiber obtained from oat bran. Further the product has the ability to increase stomach and small intestine viscosity to an extent at least as good as the uncooked ingredients of the cereal product. This enables the product to provide all the advantages of a diet high in soluble fiber yet having highly acceptable organoleptic properties. Further the product has excellent stability and long term storage properties.

The cereal product is preferably in expanded or flaked form. Also the cereal product preferably contains from about 7% by weight to about 16% by weight of β-glucan; for example from about 8% to about 12% by weight.

Preferably the cereal product comprises the oat bran concentrate and a starchy cereal flour selected from wheat flour, rice flour, corn flour, barley flour, oat flour, and rye flour; or mixtures thereof. More preferably the starchy cereal flour is wheat flour or rice flour, or a mixture thereof.

The cereal product preferably contains at least 70% by weight of the oat bran concentrate; and more preferably at least 75% by weight. The oat bran concentrate preferably has a β-glucan content of greater than about 14% by weight and more preferably from 15% by weight to about 20% by weight; especially from about 16% by weight to about 18% by weight. The total dietary fiber content of the oat bran concentrate is preferably above 25% by weight, more preferably in the range of about 30% by weight to about 40% by weight; for example from about 32% by weight to about 36% by weight.

Preferably the oat bran concentrate contains less than 7% by weight of oils and fats; more preferably less than 6% by weight. For example, the oat bran concentrate may contain from about 4% by weight to about 6% by weight of fats and oils.

In another aspect, this invention provides a ready-to-eat food product comprising an extrusion cooked cereal product as defined above.

The food product may be a breakfast cereal comprising the extrusion cooked cereal product in flaked or expanded form, or both, the extrusion cooked cereal product being coated with a fructose coating. Preferably the food product comprises more than 50% by weight of the extrusion cooked cereal product; more preferably from 60 to 80% by weight of the extrusion cooked cereal product.

The food product may be a cereal bar comprising the extrusion cooked cereal product in flaked or expanded form, or both, and a granola component selected from nuts, dried fruit, grains, expanded products, and mixtures thereof. Preferably the cereal bar has a saturated fat content such that the saturated fat provides less than about 15% of total calories. More preferably the saturated fat provides less than about 10% of total calories. Preferably the cereal bar has a content of mono-unsaturated fats such that the mono-unsaturated fats provide from about 5% to about 20% of total calories.

In another aspect, the invention provides a method for the prophylaxis or treatment of diabetes, the method comprising orally administering to a patient an extrusion cooked cereal product containing a defatted oat bran concentrate which has a β-glucan content of at least 10% by weight and which in solution provides a viscosity at least as great as the viscosity provided by the components of the cereal product prior to being extrusion cooked; the cereal product being administered in an amount sufficient to provide a daily dose of β-glucan of about 1 g to about 25 g. The method is preferably for the prophylaxis or treatment of type II diabetes.

In a further aspect, this invention provides the use of a defatted oat bran concentrate which has a β-glucan content of at least 10% by weight in the production of an extrusion cooked cereal product which in solution provides a viscosity at least as great as the viscosity provided by the components of the cereal product prior to being extrusion cooked, for the prophylaxis and treatment of diabetes; preferably type II diabetes.

Preferably, the cereal product is administered in the form of a ready-to-eat breakfast cereal or a cereal bar.

### Brief Description of the Drawings

Embodiments of the invention are now described, by way of example only, with reference to the drawings in which:

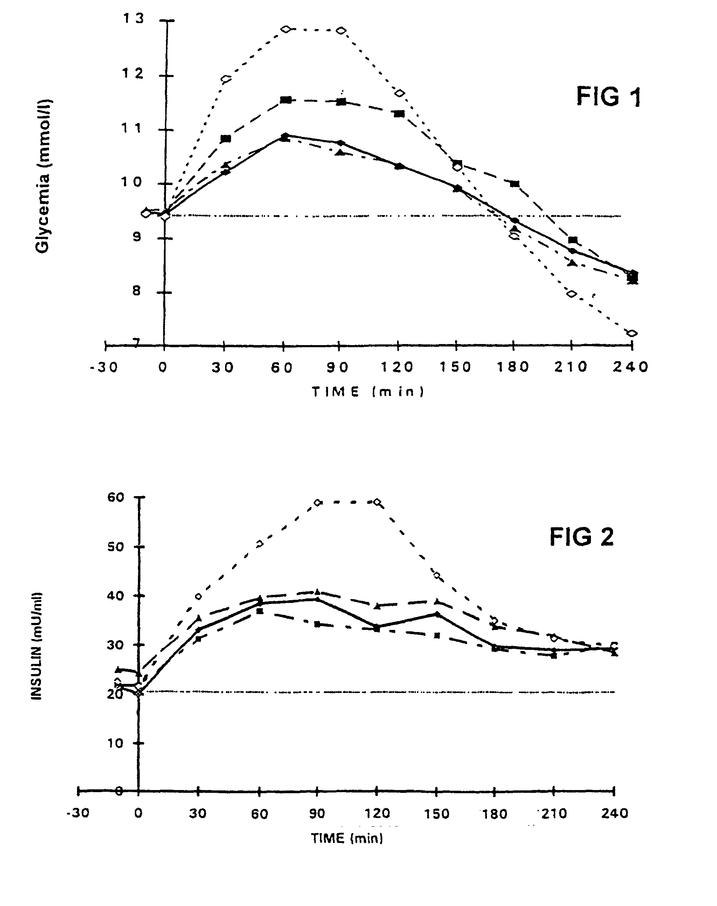

Figure 1 is a graph of mean plasma glucose level (glycemia) against time after consumption of one of four breakfasts:- breakfast 1 (0 g of soluble fiber) is indicated as --◇--, breakfast 2 (4 g of soluble fiber) is indicated as --■--, breakfast 3 (6 g of soluble fiber) is indicated as ―◆―, and breakfast 4 (8.4 g of soluble fiber) is indicated as --Δ--.
Figure 2 is a graph of mean plasma insulin level against time after consumption of one of four breakfasts:- breakfast 1 (0 g of soluble fiber) is indicated as --◇--, breakfast 2 (4 g of soluble fiber) is indicated as --Δ--, breakfast 3 (6 g of soluble fiber) is indicated as ―◆―, and breakfast 4 (8.4 g of soluble fiber) is indicated as --■--.

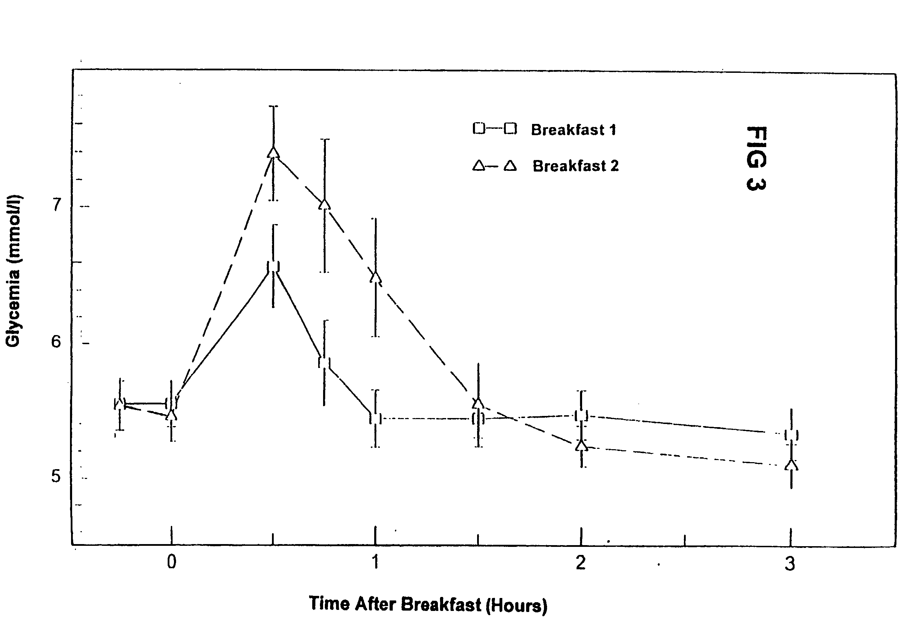

Figure 3 is a graph of mean plasma glucose level (glycemia) against time after consumption of one of two breakfasts:- breakfast 1 is indicated as ―□― and breakfast 2 is indicated as --Δ--.

### Detailed Description of the Invention

To produce the extrusion cooked cereal product, an uncooked dry mix of the dry ingredients of the cereal product is prepared. The primary dry ingredient of the dry mix is a defatted oat bran concentrate which preferably has a total fiber content of at least 30% by weight and a β-glucan content of at least 14% by weight. The dry mix will comprise at least 50% by weight of the oat bran concentrate; the exact amount depending upon the desired form and desired properties of the cereal product. In this specification, a "defatted oat bran concentrate" means an oat bran fraction which has an enriched soluble fiber content of above about 10% by weight and which has been subjected to solvent extraction to remove, at least partially, oils and fats from the fraction. Ordinarily, oat bran concentrates have a fat or oil content of greater than about 10% by weight. Defatted oat bran concentrates have an oil or fat content of less than about 7% by weight; more usually about 4% to about 6% by weight.

Defatted oat bran concentrates of this type are commercially available; for example suitable oat bran concentrates may be purchased from Swedish Protein AB, Väröbacka, Sweden. Alternatively the oat bran concentrate may be prepared by grinding dry oat grains and then carefully screening the fiber material from the starchy components of the oat grains. The fiber rich material may then be subjected to solvent extraction techniques to remove oils and fats from the material. A suitable procedure for the extraction of oils and fats is disclosed in British patent 1,526,553; the disclosure of which is incorporated by reference. The solvent extraction step may also be carried out prior to screening if desired. This screening and extraction procedure would be suitable for producing oat bran concentrates with fiber contents at the lower end of the range; for example an oat bran concentrate having a maximum soluble fiber content of about 15% by weight.

As another alternative, the process described in US patent 5,106,640 (the disclosure of which is incorporated by reference) may be used to produce the defatted oat bran concentrate. In this process, oat grains are rapidly ground in slurry form at a temperature of 0 to 15°C. The slurry is then homogenized and then screened to separate off a fiber enriched fraction. The fiber enriched fraction is then subjected to extraction to remove oils and fats. Using this technique, oat bran concentrates having β-glucan contents of up to about 40% by weight may be prepared. Although oat bran concentrates having very high β-glucan contents may be used to produce the cooked and expanded cereal product, it is preferred if the β-glucan content is less than about 20% by weight.

If it is desired to produce an expanded cereal product, the dry mix will usually also contain a starchy, farinaceous ingredient or a starch, or both. Any suitable starchy farinaceous ingredient may be used. Suitable examples are wheat flour, rice flour, corn flour, barley flour, oat flour, and rye flour. Also mixtures of these flours may be used. The flours may be whole flours or may be flours which have had fractions removed; for example the germ fraction or husk fraction may be removed. Rice flour and wheat flour are particularly suitable; either alone or in combination. For example, up to 40% by weight of the starchy, farinaceous ingredient or starch may be used although adequate expansion may be obtained with lower amounts of the starchy, farinaceous ingredient or starch.

The amount of the starchy, farinaceous ingredient or starch that is used is selected to provide the desired properties in the end product but should not be such that the β-glucan content of the cereal product drops below 5%. If it is desired to provide a highly expanded product, greater amounts of the starchy, farinaceous ingredient or starch will be needed. In this case, the β-glucan content of the end product may be maintained above 5% by weight by using an oat bran concentrate with a high β-glucan content. However, for a oat bran concentrate which has a β-glucan content in the range of 15 to 20% by weight, the starchy, farinaceous ingredient or starch conveniently comprises from about 10% to about 30% by weight of the dry mix.

If it is desired to produce a flaked cereal product, it is not needed to use a starchy, farinaceous ingredient or a starch in the dry mix since it is possible to flake an unexpanded product. In any event however, subjecting a dry mix which comprises almost exclusively the oat bran concentrate will result in some expansion. Extensively expanded products may also be flaked.

The dry mix may also include minor amounts, for example less than 5% by weight in total, of flavoring agents, coloring agents, salts, antioxidants, vitamins, minerals, protein sources, malts, and the like. Suitable protein sources are milk powders, whey powders, wheat glutens etc. If desired, sources of insoluble fiber may also be included; for example wheat bran, corn bran, rice bran, rye bran and the like.

The various ingredients of the dry mix are then mixed to provide a homogeneous mix and are then fed into an extruder-cooker. Any suitable extrusion cooker may be used; single crew or twin screw. Suitable extrusion cookers may be commercially obtained; for example the Wenger and Clextral extrusion cookers which are widely available and well known in the art. Twin screw extruders which have co-rotating and inter-penetrating screws are particularly suitable.

Water is also fed into the extruder-cooker, usually into the second zone of the extruder-cooker, to enable gelatinization of starch components. The water may be at a temperature of about 20°C to about 60°C. The amount of water used may be selected as desired but preferably makes up less than about 25% by weight of the total weight of the water and dry ingredients. If too much water is used, it is found that the end product becomes very hard and dense. Although this may not be too serious if the end product is to be flaked, it is best avoided if the end product is to used in expanded form. For expanded cereal products, the amount of added water used is preferably less than 15% by weight of the total weight of the water and dry ingredients; for example about 10% by weight. Steam may also be used in place of water.

If desired, a very small amount of an edible oil may be fed into the extruder-cooker to facilitate the extrusion process or as carriers for oil soluble additives. Any suitable oil may be used; for example vegetable oils such as sunflower oil, safflower oil, corn oil, and the like. If oils are used, oils which are high in mono-unsaturates are particularly preferred. Hydrogenated oils or fats are also preferred. The amount of oil used is preferably kept below about 1% by weight of the mixture of oil, dry mix and water.

The rotational speed of the screw or screws is preferably kept below about 500 rpm. Above about 500 rpm, it is found that the soluble fibers are degraded due to the high shear and the end cereal product produces lower viscosities in the stomach and small intestine. Rotational speeds in the range of about 200 rpm to about 450 rpm are suitable; particularly rotational speeds in the range of 250 to 350 rpm.

The pressure in the shear and compression zones of the extruder-cooker is preferably kept below about 200 bars; for example a pressure in the range of about 100 to about 180 bars would be suitable. Particularly advantageous are pressures in the range of about 120 bar to about 140 bar.

The maximum product temperature in the extruder is preferably kept below about 200°C; for example in the range of about 100°C to about 200°C. Particularly preferred are maximum product temperatures in the range of about 120°C to about 190°C; for example about 150°C to about 180°C.

Upon leaving the extruder-cooker, the cooked cereal product is conveniently cut into small pieces using rotating blades at the exit. Depending upon the conditions in the extruder-cooker, the cooked cereal product expands to a greater or lesser extent. The cooked cereal products intended to be flaked may, if desired, undergo much less expansion. Of course, it is possible to flake a highly expanded cereal product but there is no advantage in doing this.

If a flaked product is to be produced, the cooked cereal product may then be transferred to a flaking apparatus. Suitable apparatus are well known and widely used in the cereal industry and may be purchased from, for example, Buhler AG in Switzerland. If desired, the cooked cereal product may be partially dried before flaking.

The expanded cereal product or the flaked cereal product is then dried to a moisture content below about 5% by weight. This is conveniently carried out in a hot air drier as is conventional. Moisture contents of about 1% to about 3% by weight are preferred. The expanded cereal products produced in this way have a crispy, pleasant texture and good organoleptic properties. The flaked cereal products also have good texture and organoleptic properties but are less crispy. The cereal products have a pleasant taste of toasted cereal. The density of the cereal products is conveniently less than about 300 g/l.

The cooked cereal product may then be further processed as desired. For example, if the cereal product is to be used as a breakfast cereal, it may be sprayed with a syrup which contains sugars (such as fructose or glucose) or other sweeteners, coloring agents, or flavoring agents, and the like, and then dried. Then, if desired, dried fruit, nuts, other cereals, dried milk produce (such as dried yoghurt etc) may be dried mixed with or agglomerated with the coated cereal product.

Alternatively the cereal product may be formulated into convenience foods such as snack bars, cookies, biscuits, crackers, muffins and the like. Again the cereal product may be mixed with nuts, dried fruit, sugars or other sweeteners, coloring agents, or flavoring agents, and the like. To produce a snack bar, a suitable binder, for example arabic gum or gelatine, may then be added. An agent which reduces breakability of the bar may also be included; for example hydrolysed wheat. If desired, the bar may be coated with a suitable coating; for example chocolate. Processes for manufacturing snack bars are well known and are described in the art; see for example US patent 4,871,557. The saturated fat content of the food products is preferably such that the saturated fat provides less than 15% of the total calories of the food product. The mono-unsaturated fat content of the food products is preferably such that the mono-unsaturated fat provides from about 5% to about 20% of the total calories of the food product.

If sugars are included in the breakfast cereal or snack bar, fructose is preferred if the product is intended as a food for diabetics.

The cooked cereal product, when eaten, operates to lower blood glucose, insulin and cholesterol levels and hence may be used in the prevention and treatment of diabetes and antihypercholesterolemia. The cooked cereal product may also be used as a convenience breakfast or snack food. The cooked cereal product is administered orally. For the prevention and treatment of diabetes and antihypercholesterolemia, the dosage of the cooked cereal required will vary depending upon the risk and severity of the disease and on diet but may be readily set by a medical practitioner. However a daily dosage corresponding to between about 1 g of β-glucan to about 25g of β-glucan would be adequate for most purposes. A daily dosage corresponding to between about 3g of β-glucan to about 15g of β-glucan is preferred.

The cereal product may be taken in multiple doses, for example 2 to 5 times, to make up the daily dosage or may taken in a single dose. If in a single dose form, the cereal product is conveniently taken for breakfast; for example in the form of a breakfast cereal. In this case, the cereal product may be formulated such that a standard amount (for example 25 to 50 g) of cereal product provides the required dose of β-glucan. In the case of multiple doses, the cereal product may be in the form of a convenience food; for example a snack bar, or the first dose may be in the form of a breakfast cereal and the remaining doses in the form of a convenience food.

Upon being eaten, the postprandial plasma glucose levels may be 50%, or more, less than the plasma glucose levels after consumption of a meal providing an equivalent amount of carbohydrates. Plasma insulin levels may be similarly reduced. Patients having diabetes and including the cereal products as part of their diet therefore may be able to reduce other medication or eliminate the need for the other medication altogether. Similarly, patients having hypercholesterolemia may reduce plasma cholesterol levels with regular consumption of the cereal products; particularly in conjunction with a low fat, low cholesterol diet.

In the following examples, all percentages are by weight unless otherwise stated. Further, in each case viscosity is determined by milling the cereal and then suspending 8 g of the milled cereal in 100 ml of 10nM sodium phosphate buffer at pH 6.9 and at a temperature of 37°C. The starch in the suspension is then digested by adding 8 mg of pancreatin (obtained from Sigma Corporation, St Louis, MO, USA) and the suspension stirred for 1 hour at 37°C. The viscosity of the suspension is then measured using a Haake RV 12 viscometer fitted with a M150 measuring head rotated at 64 rpm.

### Example 1 Expanded Cereal

About 108 kg of a dry mixture of about 78.0% oat bran concentrate, about 10.0% rice flour, about 9.0% wheat flour, about 2% powdered malt extract, and minor amounts of salts and coloring agents is prepared. The oat bran concentrate is obtained from Swedish Protein AB, Väröbacka, Sweden and contains about 35% dietary fiber in total and about 17% soluble fiber.

The dry mixture is fed into a Clextral BC-45H extruder (screw diameter of 55 mm and a length of 600 mm) at a rate of about 79.6 kg/hr. Water is fed into the extruder at a rate of about 8.4 kg/hr. The extruder screws are rotated at 270 rpm. The temperature of the three zones of the extruder are 18°C, 69°C and 149°C. The temperature at the exit of the extruder is 184°C. The pressure in the extruder reaches 137 bar. The product leaving the extruder orifices is cut into pieces of length of about 2 to 3 mm and the pieces allowed to expand.

The pieces of expanded product are then air dried to a moisture content of about 1.6 % using air at about 120°C. The dried pieces have a crispy, pleasant texture and good mouthfeel. The dietary fiber content of the product is about 24 % and the soluble fiber content is about 13 %. The viscosity of the expanded product is determined to be greater than that of the starting dry mixture.

### Example 2 Cereal Flakes

About 105 kg of a dry mixture of about 97% oat bran concentrate, about 2% powdered malt extract, and minor amounts of salts and coloring agents is prepared. The oat bran concentrate is obtained from Swedish Protein AB, Väröbacka, Sweden and contains about 35% dietary fiber in total and about 17% soluble fiber.

The dry mixture is fed into a Clextral BC-45H extruder as described in Example 1. The rate at which the mixture is fed is about 65.5 kg/hr. Water is fed into the extruder at a rate of about 16.2 kg/hr and an edible oil mixture is fed at a rate of 0.47 kg/hr. The extruder screws are rotated at 300 rpm. The temperature of the three zones of the extruder are 18°C, 74°C and 103°C. The temperature at the exit of the extruder is 122°C. The pressure in the extruder reaches 126 bar. The product leaving the extruder orifices is cut into pieces of length of about 2 to 3 mm.

The pieces are then flaked in a flaking apparatus (obtained from Buhler AG, Switzerland) and the flakes are then dried as described in example 1. The flakes have a water content of 2.3%. The dried flakes have a crispy, pleasant texture, a dietary fiber content of about 29 % and a soluble fiber content of about 14.5 %. The flakes have a good mouthfeel. The viscosity of the expanded product is determined to be greater than that of the starting dry mixture.

### Example 3 Blood Glucose and Insulin Reduction

Three dry mixtures of oat bran concentrate (obtained from Swedish Protein AB, Väröbacka, Sweden), oat flour and starch (obtained from National Starch, Bridgewater, New Jersey, USA) are prepared. The oat bran concentrate contains 32.3% fiber including 14.9% β-glucan. The first dry mixture contains 8% β-glucan, the second dry mixture contains 10% β-glucan, and the third dry mixture contains 13% β-glucan.

The mixtures are cooked, extruded and expanded in a Clextral BC21 extruder equipped with a 500 mm barrel and twin screws of 25 mm diameter. The screw speed is set at 350 rpm and the pressure at the die varies between 140 and 150 bar. The temperature in the compression zone is about 130°C and the temperature of the product at the exit is about 122°C. The dried and expanded products have a density of about 170 to about 225 g/l. The product produced from the first dry mixture is labelled sample 3a, that from the second dry mixture is labelled sample 3b, and that from the third dry mixture is labelled sample 3c. The viscosity of sample 3a is 330 mPa, the viscosity of sample 3b is 580 mPa, and the viscosity of sample 3c is 1010 mPa.

Eight patients (7 male, 1 female) with non insulin dependent diabetes are recruited. The patients range in age from 34 years to 65 years. At the time of the trial, one patient was being treated by diet alone, 2 with metformin, and 5 with metformin and a sulfonylurea. For the trial, each patient is fasted for 10 to 12 hours overnight. In the morning, an indwelling cannula is inserted into an antecubital vein of each patient. The cannulas are kept open with a slow drip of normosaline. Blood is drawn periodically through the cannula.

Each patient is fed one of four breakfasts as follows:

| Breakfast/ Composition | | Quantity g | Energy kcal | Protein g | Lipid g | Carbohydrate /g | Fiber g | β-glucan /g |
|---|---|---|---|---|---|---|---|---|
| 1 | Bread | 66 | 143 | 6.1 | 1.7 | 28 | 5 | 0 |
| | Ham | 28 | 34 | 5.4 | 1.3 | - | - | - |
| | Jam | 30 | 7 | - | - | 2 | - | - |
| | Milk | 100 | 35 | 3.3 | 0.1 | 5 | - | - |
| | Total | 224 | 219 | 14.8 | 3.1 | 35 | 5 | 0 |
| 2 | Sample 3a | 50 | 173 | 8.5 | 3 | 27 | 9.3 | 4 |
| | Milk & Sugar | 103 | 35 | 3.3 | 0.1 | 8 | - | - |
| | Total | 153 | 208 | 11.8 | 3.1 | 35 | 9.3 | 4 |
| 3 | Sample 3b | 58 | 189 | 11.5 | 3.4 | 27 | 14.3 | 6 |
| | Milk & Sugar | 103 | 35 | 3.3 | 0.1 | 8 | - | - |
| | Total | 161 | 224 | 14.8 | 3.5 | 35 | 14.3 | 6 |
| 4 | Sample 3c | 67 | 203 | 13.6 | 4 | 27 | 18.3 | 8.4 |
| | Milk & Sugar | 103 | 35 | 3.3 | 0.1 | 8 | - | - |
| | Total | 170 | 238 | 16.9 | 4.1 | 35 | 18.3 | 8.4 |

Blood is withdrawn from each patient 10 minutes before and immediately before commencement of eating the breakfast. Thereafter blood is withdrawn every 30 minutes for the next four hours. The plasma glucose levels of each sample are determined using a Beckman Glucose Analyzer II, the plasma insulin levels are determined by radioimmune assay (Herbert *et al*; 1965; J. Clin. Endocrinol. Metab.; **25**, 1375 to 1384), and the plasma triglyceride levels are determined using a colorimetric method.

The trials are repeated another three times so that each patient eventually has all four breakfasts. The order in which the breakfasts are served is selected by latin square design.

The results for plasma glucose are presented in figure 1 and those for plasma insulin in figure 2. Breakfasts 2 to 4 have significantly reduced glucose and plasma peaks and averages compared to breakfast 1. Breakfasts 3 (10% β-glucan in the cereal) and 4 (13% β-glucan in the cereal) have much the same effect on glucose levels while all three have similar effects on insulin levels. The mean values are as follows:

| Response | Breakfast 1 | Breakfast 2 | Breakfast 3 | Breakfast 4 |
|---|---|---|---|---|
| Delta max glucose/mmol.l⁻¹ | 3.8 ± 0.4 | 2.6 ± 0.5 | 1.6 ± 0.2 | 1.5 ± 0.6 |
| AUC glucose | 6.8 ± 1.2 | 4.7 ± 1.1 | 2.8 ± 0.6 | 2.5 ± 0.6 |
| | | | | |
| Delta max insulin/mU.ml⁻¹ | 69 ± 14 | 46 ± 10 | 43 ± 10 | 35 ± 10 |

The results indicate that the improvement in the effect of the soluble fiber in reducing plasma glucose levels tails off at about 13% soluble fiber. Consequently having a concentration of soluble fiber in the cereal of above 13% would seem not to offer any advantage.

### Example 5 Breakfast Cereal

The procedure of example 1 is repeated for a dry mixture containing oat bran concentrate and wheat flour in a mass ratio of 70:30. However the extruded product leaving the extruder is flaked as described in example 2. 52 kg of the flakes are then mixed with 11.6 kg of an expanded oat bran concentrate/rice flour/wheat flour product produced substantially according to example 1 except that the mass ratio of oat bran concentrate:rice flour:wheat flour is 60:20:20. A fructose syrup made up from about 75% by weight fructose and the remainder water is sprayed over the mixture and the mixture dried. The dried mixture is then mixed with 11 kg of oat flakes and 10 kg of dried apple pieces.

The composition of the cereal is as follows:

| **Component** | **Mass percentage** |
|---|---|
| Lipids | 6% |
| Proteins | 14% |
| Carbohydrates | 60% |
| Total Fibre | 16% |
| β-glucan | 8% |

The breakfast cereal has a pleasant taste and mouthfeel; similar to conventional muesli.

### Example 6 Snack Bar

A mixture of wheat flour and rice flour is subjected to extrusion cooking using a process as described in example 1 to produced crisp, expanded balls. 13 kg of these crisp balls are mixed with 45 kg of the flaked cereal product of example 2 and 15 kg of dried raisins. A fructose syrup is prepared from fructose, arabic gum and water. 27 kg of the syrup is combined with the crisp balls, the flakes and the raisins. The mixture is formed into bars, cooled and cut into 20g portions, all in a conventional manner.

The bars have a β-glucan content of about 8% by weight, a protein content of about 12% by weight, a total fibre content of about 17% by weight, a fat content of about 4.5% by weight, and a carbohydrate content of about 56% by weight. The fat provides about 13% of total calories and is made up of about 15% by weight saturated fats, about 57% by weight mono-unsaturated fats, and about 28% by weight polyunsaturated fats. Therefore the saturated fats provide less than 2% of the total calories while the mono-unsaturated fats provide about 7.5% of total calories. The bar has good taste, texture and mouthfeel.

### Example 7 Plasma Glucose Reduction

Six healthy male volunteers of age 20 to 45 years are recruited. For the trial, each volunteer has a standardized meal the evening prior to the test and no food is taken after 10 pm. The consumption of alcohol and smoking is restricted. In the morning, each volunteer is offered one of two breakfasts as set out below:

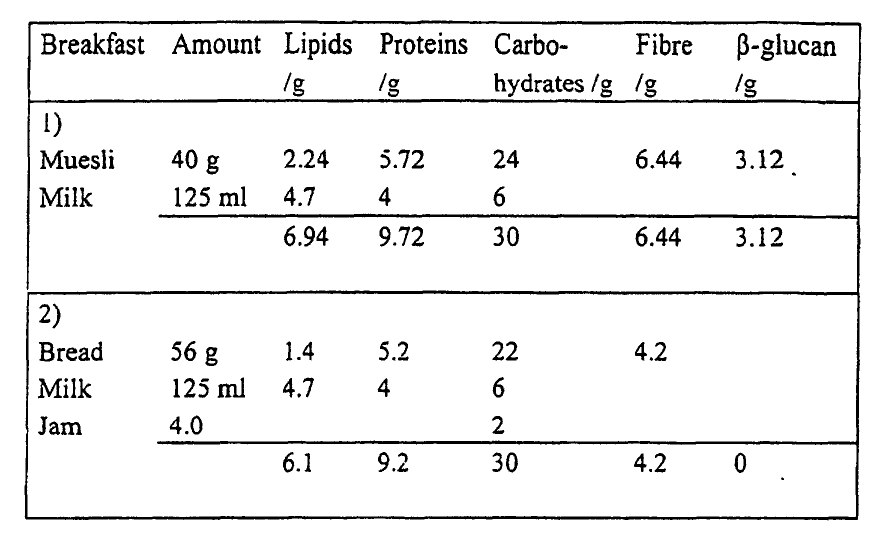

Both breakfasts have a carbohydrate content of 30 g. Breakfast 1 has an energy content of 212 kCal and breakfast 2 has an energy content of 209 kCal.

Blood is drawn from each volunteer by fingerprick 15 minutes before and immediately before commencement of eating the breakfast. Thereafter blood is drawn at 30 minutes, 45 minutes, 60 minutes, 90 minutes, 120 minutes, and 180 minutes. In each case, 100 µl are taken. The plasma glucose levels of each sample are determined using a Beckman Glucose Analyzer II. The trials are repeated again after a week so that each volunteer eventually has both breakfasts.

The results for plasma glucose are presented in figure 3. Breakfast 1 results in a glucose peak which is 50% less than that of breakfast 2. The area under the curve for Breakfast 1 is 0.7 mmol.h/l and that for breakfast 2 is 2.3 mmol.h/l.

## Claims

1. An extrusion cooked cereal product in ready-to-eat form, the cereal product containing a defatted oat bran concentrate which has a β-glucan content of at least 14% by weight and an oil or fat content of less than 7% by weight; the cereal product having a β-glucan content of 7% by weight to 16% by weight and in solution providing a viscosity at least as great as the viscosity provided by the components of the cereal product prior to being extrusion cooked.

2. A cereal product according to claim 1 further comprising a starchy cereal flour selected from wheat flour, rice flour, corn flour, barley flour, oat flour, and rye flour; or mixtures thereof.

3. A cereal product according to claim 1 or claim 2 which contains at least 60% by weight of the oat bran concentrate.

4. A cereal product according to any of claims 1 to 3 in which the oat bran concentrate has a β-glucan content of from 15% by weight to about 20% by weight.

5. A cereal product according to any of claims 1 to 5 in which the oat bran concentrate contains from 4% by weight to 6% by weight of fats and oils.

6. A ready-to-eat cereal bar for diabetics comprising:
an extrusion cooked cereal in flaked or expanded form, the cereal containing a defatted oat bran concentrate which has a β-glucan content of at least 14% by weight and an oil or fat content of less than 7% by weight and in solution providing a viscosity at least as great as the viscosity provided by the components of the cereal prior to being extrusion cooked; and
a granola component selected from nuts, dried fruit, grains, expanded products, and mixtures thereof;
the cereal bar containing sufficient of the cereal to provide at least 5% by weight of β-glucan.

7. A cereal bar according to claim 6 which contains 7% by weight to 16% by weight of β-glucan.

8. A cereal bar according to claim 6 or claim 7 which has a saturated fat content such that the saturated fat provides less than about 15% of total calories.

9. A cereal bar according to any of claims 6 to 8 which has a mono-unsaturated fat content such that the mono-unsaturated fats provide from 5% to 20% of total calories.

10. The use of a defatted oat bran concentrate which has a β-glucan content of at least 14% by weight and an oil or fat content of less than 7% by weight in the preparation of an extrusion cooked cereal product which has a β-glucan content of 7% to 16% by weight and which in solution provides a viscosity at least as great as the viscosity provided by the components of the cereal product prior to being extrusion cooked, for the prophylaxis or treatment of diabetes.

## Patentansprüche

1. Kochextrudiertes Cerealienprodukt in einer ess-fertigen Form, wobei das Cerealienprodukt ein entfettetes Haferkleiekonzentrat enthält, das einen β-Glucan-Gehalt von wenigstens 14 Gew.-% und einen Öl- oder Fettgehalt von weniger als 7 Gew.-% aufweist, wobei das Cerealienprodukt einen β-Glucan-Gehalt von 7 Gew.-% bis 16 Gew.-% aufweist und in Lösung eine Viskosität ergibt, die wenigstens so hoch ist wie die Viskosität, die man durch die Bestandteile des Getreideprodukts vor ihrer Kochextrusion erhält.

2. Cerealienprodukt nach Anspruch 1, das außerdem ein stärkehaltiges Getreidemehl umfaßt, das ausgewählt ist aus Weizenmehl, Reismehl, Maismehl, Gerstenmehl, Hafermehl und Roggenmehl oder Mischungen davon.

3. Cerealienprodukt nach Anspruch 1 oder. Anspruch 2, das wenigstens 60 Gew.-% des Haferkleiekonzentrats enthält.

4. Cerealienprodukt nach irgendeinem der Ansprüche 1 bis 3, bei dem das Haferkleiekonzentrat einen β-Glucan-Gehalt von 15 Gew.-% bis etwa 20 Gew.-% aufweist.

5. Cerealienprodukt nach irgendeinem der Ansprüche 1 bis 5, bei dem das Haferkleiekonzentrat von 4 bis 6 Gew.-% Fette und Öle enthält.

6. Essfertiger Cerealienriegel für Diabetiker, der umfasst:
eine kochextrudierte Cerealie in Flockenform oder expandierter Form, wobei die Cerealie ein entfettetes Haferkleiekonzentrat enthält, das einen β-Glucan-Gehalt von wenigstens 14 Gew.-% und einen Öl- oder Fettgehalt von weniger als 7 Gew.-% aufweist und in Lösung eine Viskosität ergibt, die wenigstens so groß ist wie die Viskosität, die von den Bestandteilen der Cerealie vor ihrer Kochextrusion erzeugt wird;
und eine körnerförmige Cerealienkomponente, die ausgewählt ist aus Nüssen, Trockenfrüchten, Körnern, expandierten Produkten und Mischungen davon;
wobei der Cerealienriegel ausreichende Mengen der Cerealie enthält, um wenigstens 5 Gew.-% β-Glucan zu liefern.

7. Cerealienriegel nach Anspruch 6, der 7 bis 16 Gew.-% β-Glucan enthält.

8. Cerealienriegel nach Anspruch 6 oder Anspruch 7, der einen solchen Gehalt an gesättigtem Fett aufweist, daß das gesättigte Fett weniger als etwa 15% der Gesamtkalorien liefert.

9. Cerealienriegel nach irgendeinem der Ansprüche 6 bis 8, der einen solchen Gehalt an mono-ungesättigtem Fett aufweist, daß die mono-ungesättigten Fette von 5 bis 20% der Gesamtkalorien liefern.

10. Verwendung eines entfetteten Haferkleiekonzentrats, das einen β-Glucan-Gehalt von wenigstens 14 Gew.-% und einen Öl- oder Fett-Gehalt von weniger als 7 Gew.-% aufweist, bei der Herstellung eines kochextrudierten Cerealienprodukts, das einen β-Glucan-Gehalt von 7 bis 16 Gew.-% aufweist und das in Lösung eine Viskosität ergibt, die wenigstens so groß ist wie die Viskosität, die von den Bestandteilen des Cerealienprodukts vor ihrer Kochextrusion erzeugt wird, zur Prophylaxe oder Behandlung von Diabetes.

## Revendications

1. Produit à base de céréales cuit par extrusion, sous une forme prête à la consommation, le produit à base de céréales contenant un concentré de son d'avoine dégraissé qui a une teneur en β-glucane d'au moins 14 % en poids et une teneur en huiles ou matières grasses inférieure à 7 % en poids ; le produit à base de céréales ayant une teneur en β-glucane de 7 % en poids à 16 % en poids et, en solution, présentant une viscosité qui est au moins aussi forte que la viscosité engendrée par les constituants du produit à base de céréales avant sa cuisson par extrusion.

2. Produit à base de céréales suivant la revendication 1, comprenant en outre une farine de céréales amylacée choisie entre la farine de blé, la farine de riz, la farine de maïs, la farine d'orge, la farine d'avoine, la farine de seigle et leurs mélanges.

3. Produit à base de céréales suivant la revendication 1 ou la revendication 2, qui contient au moins 60 % en poids du concentré de son d'avoine.

4. Produit à base de céréales suivant l'une quelconque des revendications 1 à 3, dans lequel le concentré de son d'avoine a une teneur en β-glucane de 15 % en poids à environ 20 % en poids.

5. Produit à base de céréales suivant l'une quelconque des revendications 1 à 4, dans lequel le concentré de son d'avoine contient 4 % en poids à 6 % en poids de matières grasses et d'huiles.

6. Barre aux céréales prêtes à la consommation pour sujets diabétiques, comprenant :
des céréales cuites par extrusion sous forme de paillettes ou sous forme expansée, les céréales contenant un concentré de son d'avoine dégraissé qui a une teneur en β-glucane d'au moins 14 % en poids et une teneur en huiles ou matières grasses inférieure à 7 % en poids et qui, en solution, présente une viscosité au moins aussi forte que la viscosité engendrée par les constituants des céréales avant leur cuisson par extrusion ; et
un constituant granola choisi entre des noix, des fruits secs, des céréales, des produits expansés et leurs mélanges ;
la barre aux céréales contenant suffisamment de céréales pour fournir au moins 5% en poids de β-glucane.

7. Barre aux céréales suivant la revendication 6, qui contient 7 % en poids à 16 % en poids e β-glucane.

8. Barre aux céréales suivant la revendication 6 ou la revendication 7, qui a une teneur en matières grasses saturées telle que les matières grasses saturées fournissent moins d'environ 15 % des calories totales.

9. Barre aux céréales suivant l'une quelconque des revendications 6 à 8, qui a une teneur en matières grasses mono-insaturées telle que les matières grasses mono-insaturées fournissent 5 % à 20 % des calories totales.

10. Utilisation d'un concentré de son d'avoine dégraissé qui a une teneur en β-glucane d'au moins 14 % en poids et une teneur en huiles ou matières grasses inférieure à 7 % en poids dans la préparation d'un produit à base de céréales cuit par extrusion qui a une teneur en β-glucane de 7 % à 16 % en poids et qui, en solution, présente une viscosité au moins aussi forte que la viscosité engendrée par les constituants du produit à base de céréales avant sa cuisson par extrusion, pour la prophylaxie ou le traitement du diabète.
